# FASCICULE DE BREVET EUROPEEN

(11) **EP 2 390 711 B1**
(45) Date de publication et mention de la délivrance du brevet: **10.07.2019**
(21) Numéro de dépôt: 11290222.6
(22) Date de dépôt: 11.05.2011
(51) Int. Cl.: G02C 13/00, A61B 3/02, G01S 15/88, A61B 3/032

(54) **Mesure d'une distance caractéristique de lecture d'un individu en posture naturelle de vision de près**
Messung eines charakteristischen Leseabstands einer Person in natürlicher Nahsichthaltung
Measuring a typical reading distance of an individual in a natural near-vision position

(30) Priorité: 25.05.2010 FR 1002199
(43) Date de publication de la demande: 30.11.2011
(73) Titulaire: Essilor International, 94220 Charenton-le-Pont (FR)
(72) Inventeur: Haddadi, Ahmed, 94220 Charenton-le-Pont (FR)
(74) Mandataire: Jacobacci Coralis Harle

(56) Documents cités:
- WO-A1-2008/064379
- GB-A- 2 154 332
- JP-A- 2000 325 309
- JP-A- 2005 144 084
- JP-A- 2009 160 197

## Description

### DOMAINE TECHNIQUE AUQUEL SE RAPPORTE L'INVENTION

La présente invention concerne un dispositif de mesure d'une distance caractéristique de lecture d'un individu en posture naturelle de vision de près.

Elle concerne également un procédé de mesure d'une telle distance au moyen d'un tel dispositif.

### ARRIERE-PLAN TECHNOLOGIQUE

La détermination de la distance naturelle à laquelle un individu se place par rapport à un texte pour le lire est très importante pour la réalisation de lentilles ophtalmiques procurant à leur porteur une correction optique personnalisée et particulièrement adaptée à la vision de près de l'individu.

On connait du document JP2007-097707 un dispositif qui permet de déterminer cette distance et qui comporte, d'une part, un support de lecture réalisé sous la forme d'une tablette placée à une distance adaptée à la lecture d'un texte porté par cette tablette et, d'autre part, des moyens de mesure de la distance de la tête par rapport à cette tablette. Ces moyens de mesure de distance procèdent typiquement par ultrasons.

Dans ce dispositif, l'émetteur ou le récepteur d'ultrasons est placé sur la monture de lunettes de l'individu. Ceci présente l'inconvénient d'empêcher la réalisation de la mesure dans des conditions de posture naturelle de l'individu, à savoir des conditions dans lesquelles l'individu est libre de se placer comme il le souhaite par rapport à la tablette, dans les conditions de lecture les plus confortables pour lui. On comprend en effet que la présence d'un émetteur ou d'un récepteur d'ultrasons sur sa monture de lunettes implique forcément une contrainte sur la posture de l'individu.

Une autre solution connue du document JP2000-325309 consiste à placer émetteur et récepteur d'ultrasons sur la tablette et à détecter les ultrasons émis et réfléchis sur la tête de l'individu. Cette méthode présente l'inconvénient d'être peu précise.

Dans ce dispositif connu, l'axe de mesure de l'émetteur et du récepteur est sensiblement perpendiculaire au plan de la partie de lecture de la tablette. Pour que la mesure puisse être réalisée, il est donc nécessaire de placer la tablette de telle manière que l'axe de mesure de l'émetteur et du récepteur pointent en direction de l'individu et, partant, que le plan de la partie de lecture de la tablette soit orienté face à lui. Or, cette configuration ne correspond pas à une posture de lecture naturelle.

On connaît enfin du document JP2005-144084 un dispositif de mesure d'une distance entre le dispositif et la tête d'un individu comportant un support de lecture avec une partie d'affichage plane et un sous-ensemble de mesure comportant des moyens de mesure de distance par ultrasons.

On connait également du document WO2008/064379 un dispositif conforme au préambule de la revendication 1.

### OBJET DE L'INVENTION

Un but de la présente invention est de proposer un dispositif et une méthode de mesure de la distance de lecture en vision de près d'un individu dans sa posture naturelle, qui soit rapide, précise et facile à mettre en oeuvre, avec aussi peu de contrainte que possible sur la posture de l'individu.

A cet effet, on propose selon l'invention un dispositif de mesure d'une distance caractéristique de lecture d'un individu en posture naturelle de vision de près, tel qu'énoncé dans la revendication 1.

Le dispositif selon l'invention permet de laisser l'individu dont on souhaite mesurer la distance caractéristique de lecture placer la partie d'affichage du support de lecture sur laquelle il va lire des signes à une distance librement choisie par rapport à ses yeux et en particulier par rapport aux lentilles ophtalmiques de sa monture de lunettes, afin de réaliser une mesure dans des conditions de posture naturelle de l'individu.

Dans la posture naturelle, le plan de cette partie d'affichage est incliné devant le visage de l'individu.

La mesure est effectuée selon un principe classique de mesure de distance par ultrasons : l'organe récepteur détecte des signaux ultrasons émis par l'émetteur et réfléchis sur la tête de l'individu ou plus précisément sur un accessoire disposé sur sa tête. En pratique, il est particulièrement avantageux de réaliser cette mesure par ultrasons car la différence de réflectivité entre l'accessoire typiquement monture et lentilles ophtalmiques d'une paire de lunettes disposée sur la tête de l'individu et la peau de l'individu permet de distinguer facilement le signal réfléchis par l'accessoire.

La distance caractéristique de lecture mesurée est donc de préférence la distance entre la monture de lunettes de l'individu et le dispositif de mesure.

Afin de réaliser la mesure aisément, il est avantageux d'utiliser un organe émetteur d'ultrasons émettant dans un cône de mesure présentant un angle au sommet suffisamment large pour couvrir facilement l'ensemble de la tête de l'individu ou du moins l'accessoire équipant la tête de l'individu. L'angle au sommet du cône de mesure de l'organe émetteur sera ainsi supérieur ou égal à 15 degrés.

Afin de réaliser une mesure précise, il est avantageux d'utiliser un premier organe de mesure émettant ou recevant les ultrasons exclusivement dans un cône de mesure d'angle au sommet inférieur ou égal à 45 degrés.

Il est alors utile de vérifier que la tête de l'individu se trouve bien dans ce cône de mesure.

Plus particulièrement, afin d'optimiser le signal d'ultrasons mesuré par le dispositif de mesure, il est alors préférable d'orienter l'axe de mesure du premier organe de mesure sensiblement perpendiculairement au plan moyen de la monture de l'individu.

Cette orientation de l'axe de mesure du premier organe de mesure permet avantageusement de réaliser une mesure précise, notamment par la mesure d'un signal présentant un rapport signal sur bruit satisfaisant.

L'organe de mesure comprend un élément de mesure émetteur, récepteur ou émetteur-récepteur placé dans un corps. On entend ici par axe de mesure un axe selon lequel l'émission ou la réception par le premier organe de mesure est privilégiée. Pour un organe récepteur, cet axe de mesure correspond à l'axe passant par l'élément récepteur selon lequel l'intensité du signal ultrasons reçu est maximale lorsque cet axe pointe vers une source d'ultrasons. Pour un organe émetteur, cet axe de mesure correspond à l'axe passant par l'élément émetteur selon lequel l'intensité du signal ultrasons émis est maximale lorsque cet axe pointe vers un élément récepteur d'ultrasons.

Dans le cas d'un organe émetteur-récepteur, cet axe de mesure correspond à l'axe selon lequel un signal d'intensité maximale est détecté pour un signal émis d'intensité déterminée.

En pratique, l'axe de mesure correspond à un axe de symétrie du corps de l'organe de mesure. Ce corps présente par exemple typiquement la forme d'une cavité cylindrique ou conique au fond de laquelle est placé l'élément de mesure. L'axe de mesure correspond alors à l'axe longitudinal de cette cavité cylindrique ou à l'axe de révolution de cette cavité conique.

Le cône de mesure de l'organe de mesure désigne alors un angle solide qui représente l'ensemble des axes de propagation des ultrasons de l'espace selon lesquelles les ultrasons sont émis ou reçus par l'organe de mesure. Cet angle solide est centré sur cet axe de mesure et correspond sensiblement à l'angle solide délimité par la cavité conique.

En posture naturelle de lecture pour l'individu, la partie d'affichage du dispositif n'est pas orientée parallèlement au plan moyen de la monture de l'individu. Typiquement, en posture naturelle, la direction perpendiculaire au plan de la partie d'affichage ne pointe pas en direction de la tête ou des lunettes de l'individu.

Il serait donc impossible de réaliser la mesure de distance entre le dispositif et les montures de l'individu si l'axe de mesure de l'organe de mesure était orienté selon cette direction.

Dans cette situation, l'individu serait obligé de fortement incliner le dispositif, et donc sa partie d'affichage, par rapport à sa position dans la posture naturelle de lecture afin de réaliser la mesure.

Selon l'invention, l'axe de mesure étant inclinable au moins d'un angle compris entre 10 et 80 degrés, la modification d'inclinaison de la partie d'affichage du dispositif nécessaire pour amener cet axe de mesure selon la direction perpendiculaire au plan des lentilles ophtalmiques de la monture de lunettes de l'individu est limitée. Elle entraîne ainsi soit une modification limitée de l'angle d'inclinaison de la partie d'affichage, soit aucune modification de cet angle d'inclinaison de la partie d'affichage.

Selon l'invention, le premier organe de mesure est monté mobile sur le support de lecture pour au moins pivoter par rapport au plan de ladite partie d'affichage du support de lecture.

Le pivotement du premier organe de mesure par rapport à cette partie d'affichage permet alors une orientation de l'axe de mesure du premier organe de mesure indépendamment de la position et de l'orientation spatiale de la partie d'affichage du support de lecture. Cette partie d'affichage peut ainsi conserver le positionnement que lui a fait prendre l'individu et qui correspond à la posture de lecture naturelle de ce dernier alors que la position angulaire de l'axe de mesure du premier organe de mesure est ajustée pour assurer que la tête de l'individu entre dans le cône de mesure de cet organe et, de préférence, que son axe de mesure se trouve perpendiculaire au plan moyen de la monture de l'individu.

Ce plan moyen de la monture correspond en première approximation au plan moyen des lentilles ophtalmiques montées dans cette monture.

L'inclinaison du dispositif et la distance de lecture choisies par l'individu restent alors tout à fait naturelles pour la mesure de la distance caractéristique de lecture.

Cette capacité de pivotement du premier organe de mesure permet d'optimiser finement le signal d'ultrasons détecté et en conséquence d'améliorer la précision de la mesure de distance réalisée.

D'autres caractéristiques avantageuses et non-limitatives du dispositif selon l'invention sont énoncées dans les revendications 2 à 7.

L'invention concerne également un procédé de mesure d'une distance caractéristique de lecture d'un individu en posture naturelle de vision de près.

Selon l'invention, le procédé comporte les étapes décrites dans la revendication 8.

Le réglage de l'inclinaison de l'axe de mesure du premier organe de mesure est plus fin et le positionnement de la partie d'affichage est totalement libre.

La mesure réalisée selon ce mode de réalisation perfectionné du procédé correspond donc à une posture de l'individu la plus proche possible de la posture naturelle.

D'autres caractéristiques du procédé selon l'invention sont décrites dans les revendications 9 à 13.

### DESCRIPTION DETAILLEE D'UN EXEMPLE DE REALISATION

La description qui va suivre, en regard des dessins annexés, donnée à titre d'exemple non limitatif, fera bien comprendre en quoi consiste l'invention et comment elle peut être réalisée.

Sur les dessins annexés :
- la figure 1 est une vue en perspective d'un exemple de réalisation du dispositif de mesure selon l'invention ;
- la figure 2 est une vue schématique de dessus de la tête de l'individu et du sous-ensemble de mesure du dispositif de la figure 1, disposé en regard de la face de l'individu ;
- la figure 3 est une vue schématique de profil de la tête de l'individu et d'une partie du dispositif de mesure, illustrant le principe du centrage visuel par parallaxe d'une cible et d'un marquage associés à un organe de mesure du sous-ensemble de mesure pour le réglage de l'orientation de l'axe de mesure du premier organe de mesure par rapport à la tête de l'individu, pour placer cet axe de mesure dans une position angulaire pour laquelle l'organe récepteur capte les ultrasons issus de l'organe émetteur et réfléchis par la tête de l'individu ou par un accessoire disposé sur celle-ci ;
- les figures 4A, 4B et 4C sont des vues de détail de face montrant respectivement trois variantes de réalisation de la cible et du marquage lorsqu'ils sont centrés, c'est-à-dire lorsque les organes émetteur et récepteur pointent en direction du visage de l'individu ;
- la figure 5 est une vue schématique de profil de la tête de l'individu par rapport au sous-ensemble de mesure du dispositif de la figure 1.

Dans la description qui suit, l'opticien détermine une distance de lecture caractéristique d'un individu.

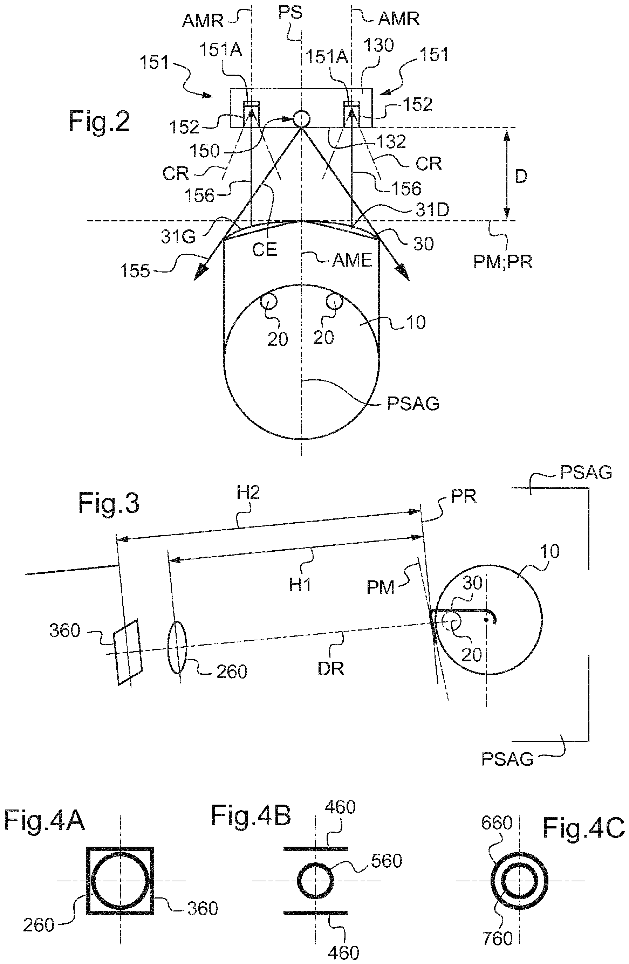

Comme le montrent les figures 2, 3 et 5, la tête 10 de l'individu est équipée d'un accessoire 30, qui est de préférence une monture 30 de lunettes (voir figure 2) munie de lentilles ophtalmiques de correction visuelle 31D, 31G.

L'individu est dans une configuration assise ou debout.

On définit ici la direction verticale comme la direction donnée par un fil à plomb en un lieu déterminé. Une direction horizontale est perpendiculaire à cette direction verticale.

Un plan sagittal PSAG de la tête de l'individu est un plan médian de la tête de l'individu passant par le milieu des deux centres de rotation des yeux 20 et perpendiculaire à la droite reliant ces deux centres de rotation des yeux 20 (figure 2). Le plan sagittal est sensiblement vertical lorsque l'individu est assis ou debout en posture naturelle.

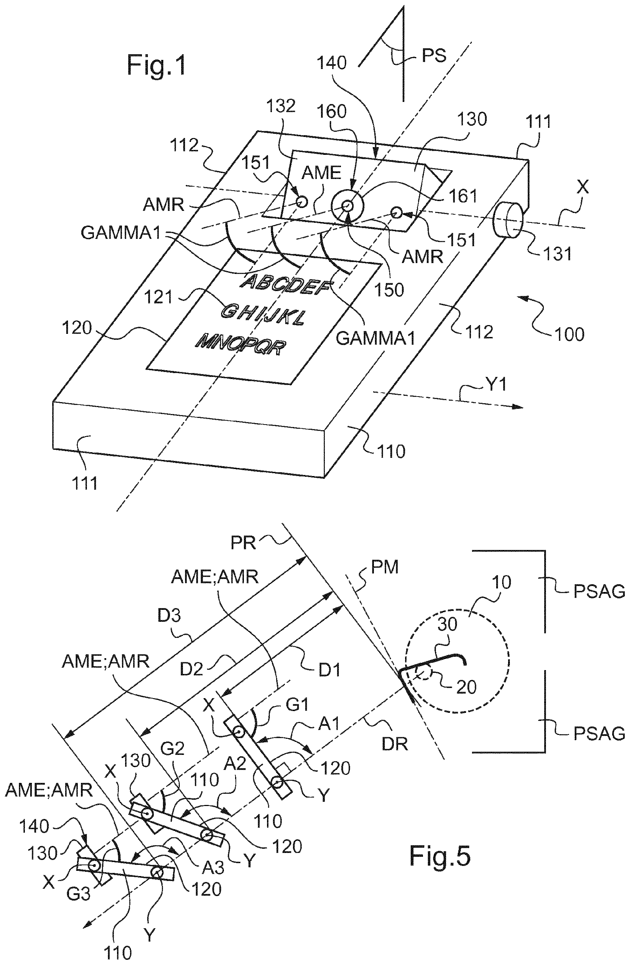

On définit une direction de regard DR comme la droite bissectrice de l'angle entre les droites reliant chaque oeil de l'individu au point fixé du regard par l'individu (figures 3 et 5).

On définit également un plan de regard PR perpendiculaire à la direction de regard DR (figures 2, 3 et 5). En première approximation, le plan de regard PR est confondu avec le plan moyen PM de la monture 30, comme représenté sur la figure 2.

Le plan moyen PM de la monture 30 est ici confondu avec les plans moyen des lentilles ophtalmiques correspondantes.

On a représenté sur la figure 1 un mode de réalisation du dispositif de mesure 100 selon l'invention.

Ce dispositif de mesure 100 comporte ici un support de lecture sous la forme d'une tablette 110 portable ou librement mobile.

La tablette 110 présente ici une forme globalement rectangulaire délimitée par des bords transversaux 111 et des bords longitudinaux 112 parallèles et opposés. En variante, on peut prévoir qu'elle présente une forme quelconque.

Cette tablette 110 comporte en face avant, c'est-à-dire du côté destiné à être orienté vers la tête 10 de l'individu, une partie d'affichage 120 plane sur laquelle sont affichés des signes 121 que l'individu fixe du regard lors de la mesure.

Les signes 121 sont typiquement alignés suivant plusieurs lignes d'affichage et comportent par exemple des optotypes semblables à ceux utilisés par l'opticien pour évaluer le défaut visuel de l'individu. Il peut aussi s'agir d'un texte quelconque que l'individu lit.

Cette partie d'affichage 120 peut comporter des signes fixes ou un écran d'affichage dynamique autorisant l'affichage de différents signes, par exemple autorisant l'affichage de textes dans lesquels les lettres utilisées présentent différentes hauteurs.

La tablette 110 est de préférence portable et l'individu la tient entre ses mains pendant la mesure.

Cependant, il peut être utile de rendre cette tablette inamovible, par exemple pour éviter le vol de la tablette dans la boutique de l'opticien.

Dans ce cas, la tablette est montée par exemple sur un bras permettant le réglage de la position de la tablette dans l'espace devant l'individu, notamment en ce qui concerne la distance entre la tablette et la tête de l'individu dans un plan horizontal et la hauteur entre la tablette et la tête de l'individu dans un plan vertical ainsi que l'inclinaison de la tablette autour d'un axe horizontal.

L'individu peut alors régler à la main la position de la tablette en faisant varier ces paramètres ou faire ces réglages de manière télécommandée, ou encore donner à un opérateur les instructions nécessaires pour que la tablette soit placée dans une position de lecture confortable pour cet individu.

En variante, la tablette peut également être posée sur une table, cette table étant adaptée à être déplacée pour que l'individu se place en posture naturelle de lecture par rapport à la table.

Dans tous les cas, l'individu ajuste la position relative de sa tête par rapport à la tablette pour se trouver dans une position naturelle et confortable de lecture. La tablette 110 est alors dans une position de lecture convenant à l'individu.

On suppose que, dans cette position de lecture naturelle et confortable de l'individu, celui-ci maintient le plan sagittal PSAG de sa tête vertical.

On suppose également qu'afin de lire le texte ou de visualiser les signes de la partie d'affichage 120 l'individu place de préférence cette partie d'affichage 120 sensiblement en face de lui.

La partie d'affichage 120 est située de préférence dans une zone médiane de la tablette 110 de sorte que la tablette 110 s'étend de part et d'autre dudit plan sagittal PSAG de la tête de l'individu.

Enfin, dans cette position de lecture naturelle et confortable de l'individu, celui-ci oriente préférentiellement le texte ou les signes à lire horizontalement. Il fait donc pivoter la tablette 110 autour d'un axe horizontal Y1 (figure 1) parallèle au texte de la partie d'affichage 120. En outre, l'individu maintient la partie d'affichage du texte à lire dans un plan sensiblement perpendiculaire au plan sagittal de sa tête (figure 2).

Alternativement, on peut prévoir que l'individu ajuste également l'orientation de la tablette autour d'un axe perpendiculaire à cet axe horizontal et parallèle au plan de la partie d'affichage. Ceci correspond à un mouvement de basculement du dispositif de mesure de gauche à droite ou de droite à gauche

Dans le cas d'une tablette 110 globalement rectangulaire, comme dans les exemples de réalisation de la figure 1, les signes 121 ou le texte à lire de la partie d'affichage sont donc de préférence orientés parallèlement à deux bords opposés de la tablette 110 ici les bords transversaux 111 .

La manipulation de la tablette 110 par l'individu se fait ainsi de manière plus facile.

Le dispositif de mesure 100 comporte en outre un sous-ensemble de mesure 140 comportant différents organes de mesure, dont au moins un organe émetteur et au moins un organe récepteur d'ultrasons.

Comme cela est expliqué plus en détail ci-après, le dispositif de mesure peut comporter soit au moins un organe émetteur et au moins un organe récepteur d'ultrasons séparés et distincts, soit au moins un seul organe à la fois émetteur et récepteur d'ultrasons.

De préférence, le sous-ensemble de mesure 140 et la partie d'affichage 120 de la tablette 100 présente un plan de symétrie PS commun.

Ce plan de symétrie PS est sensiblement confondu avec le plan sagittal PSAG de la tête 10 de l'individu dans la position de lecture.

Selon le mode de réalisation de l'invention représenté sur la figure 1, ledit sous-ensemble de mesure 140 comporte par exemple un seul organe émetteur 150 et deux organes récepteurs 151.

Ces organes de mesure 150, 151 sont tous trois portés par la tablette 110. Ils sont ici alignés selon une droite parallèle aux lignes d'affichage des signes 121 de la partie d'affichage 120 et donc parallèle aux bords transversaux 111 de la tablette 110.

Plus précisément, l'organe émetteur 150 est disposé au milieu des organes récepteurs 151. Ces deux organes récepteurs 151 sont ici distants de 7 centimètres. Cet écart permet de placer chaque organe récepteur en vis-à-vis de l'une des lentilles ophtalmiques 31D, 31G de la monture 30.

De manière remarquable, les organes émetteur 150 et récepteur 151 sont portés par la tablette 110 et l'un au moins de ces organes émetteur 150 et récepteur 151 possède un axe de mesure AMR, AME, selon lequel l'émission ou la réception est privilégiée qui est inclinable par rapport au plan de la partie d'affichage 120 au moins dans une plage angulaire comprise entre 10 et 80 degrés.

L'organe émetteur 150 ou récepteur 151 en question est plus précisément monté mobile sur la tablette 110 pour au moins pivoter par rapport à ladite partie d'affichage 120 de celle-ci.

Plus particulièrement, dans l'exemple représenté sur les figures, les trois organes émetteurs 150 et récepteurs 151 sont montés mobiles sur la tablette 110.

Ils sont montés pour pivoter tous les trois conjointement sur la tablette 110. Les axes de mesure AME, AMR1, AMR2 de ces organes émetteurs 150 et récepteurs 151 sont alors tous inclinés d'un même angle GAMMA1 par rapport au plan de la partie d'affichage 120.

Selon une variante particulièrement avantageuse de ce mode de réalisation perfectionné, le sous-ensemble de mesure comporte deux organes à la fois émetteurs et récepteurs d'ultrasons, et au moins l'un de ces organes émetteur-récepteur possède un axe de mesure selon lequel l'émission ou la réception est privilégiée qui est inclinable par rapport au plan de la partie d'affichage au moins dans une plage angulaire comprise entre 10 et 80 degrés.

Ces deux organes émetteur-récepteur sont de préférence distants de 7 centimètres de sorte qu'ils soient placé chacun en vis-à-vis de l'une des lentilles ophtalmiques de la monture.

Selon une autre variante, le sous-ensemble de mesure comporte un seul organe émetteur-récepteur central. Le fonctionnement du dispositif de mesure reste le même.

Le sous-ensemble de mesure 140 comporte ici un support de mesure 130 qui est monté pour pivoter sur la tablette 110 et qui porte les organes émetteurs 150 et récepteurs 151.

Le support de mesure 130 est monté à pivotement autour d'un axe de pivotement X parallèle à la droite d'alignement de l'émetteur 150 et des récepteurs 151, donc perpendiculaire au plan de symétrie PS du sous-ensemble de mesure 140 et de la partie d'affichage 120.

Le support de mesure 130 est par exemple adapté à être incliné au moins dans une plage d'inclinaison dans laquelle l'angle GAMMA1 est compris entre 10 et 80 degrés.

De préférence, ce support de mesure 130 pivote d'un angle au moins égal à 15 degrés vers l'avant ou vers l'arrière de la tablette 110 par rapport à une position angulaire moyenne de référence dans laquelle l'angle GAMMA1 est compris entre 10 et 80 degrés.

Lorsque la tablette 110 est placée par l'individu dans la position de lecture, cet axe de pivotement X est maintenu sensiblement horizontal.

Ici le pivotement du support de mesure 130 autour de l'axe de pivotement X est commandé manuellement par l'individu à l'aide d'une molette 131.

En variante, le support de mesure peut être monté sur la tablette par l'intermédiaire d'une charnière, et l'individu peut faire pivoter manuellement ce support en agissant directement sur lui. Il n'est pas alors prévu de molette.

Ainsi, comme représenté sur la figure 5, quel que soit l'angle d'inclinaison A1, A2, A3 de la partie d'affichage 120 par rapport à la droite de regard DR dans le plan sagittal PSAG de la tête de l'individu dans la position de lecture, il est possible de faire pivoter le sous-ensemble de mesure 140 autour de l'axe de pivotement X pour orienter les organes émetteurs 150 et récepteurs 151 vers la tête de l'individu.

Il est alors possible de positionner précisément les organes émetteurs 150 et récepteurs 151 du sous-ensemble 140 par rapport à la tête de l'individu afin d'optimiser le signal d'ultrasons détecté, c'est-à-dire pour obtenir un signal d'ultrasons d'intensité maximum ou supérieure à un seuil.

Pour cela, comme représenté sur la figure 5 et expliqué plus en détail ci-après, les axes de mesure AME, AMR des organes émetteur 150 et récepteur 151 du sous-ensemble de mesure 140 sont inclinés respectivement d'un angle G1, G2, G3 par rapport à la partie d'affichage 120 lorsque celle-ci est inclinée d'un angle égale à A1, A2 et A3, de telle sorte que les axes de mesure AME, AMR des organes émetteurs 150 et récepteurs 151 restent perpendiculaires au plan de la monture PM confondu avec le plan de regard PR quel que soit l'angle d'inclinaison de la partie d'affichage 120 (figure 5).

Selon un principe classique de mesure de distance par ultrasons, les organes récepteurs 151 détectent des signaux ultrasons émis par l'émetteur 150 et réfléchis sur la tête 10 du porteur (voir figure 2).

Le dispositif de mesure 100 comporte des moyens électroniques et informatiques (non représentés) de traitement du signal d'ultrasons détecté par les organes récepteurs.

Les ultrasons sont réfléchis d'autant plus efficacement que la surface sur laquelle ils se réfléchissent est dure. Le signal d'ultrasons réfléchis sur la monture 30 de lunettes et les lentilles ophtalmiques 31D, 31G montées sur cette monture 30 est donc bien supérieur au signal d'ultrasons correspondant aux ultrasons réfléchis sur la peau de l'individu. La détection de la position de la monture 30 de l'individu munie de ses lentilles ophtalmiques est donc à la fois facile et précise, puisque le signal d'ultrasons détecté est plus important et moins soumis à des variations dues à des signaux parasites.

La distance de lecture caractéristique de l'individu mesurée est donc ici de préférence la distance séparant l'organe émetteur 150 de la tablette 110 des lunettes 30 de l'individu.

Cette distance est notée D sur la figure 2.

La figure 5 montre trois positions de lecture possibles pour la tablette 110. La première position correspond à une distance de lecture D1 et un angle d'inclinaison A1 de la tablette 110 par rapport à la direction de regard DR. La deuxième position correspond à une distance de lecture D2 et un angle d'inclinaison A2 de la tablette 110 par rapport à la direction de regard DR. La troisième position correspond à une distance de lecture D3 et un angle d'inclinaison A3 de la tablette 110 par rapport à la direction de regard DR.

A titre d'exemple, la distance D1 est égale à 25 cm et l'angle A1 est égale à 90 degrés. La distance D2 est égale à 40 cm et l'angle A2 est égale à 110 degrés. La distance D3 est égale à 50 cm et l'angle A3 est égale à 130 degrés.

Dans le mode de réalisation perfectionné représenté sur les figures 1, 2 et 5, on utilise de préférence un organe émetteur 150 émettant des ultrasons dans un angle solide large, présentant par exemple un angle au sommet supérieur ou égal à 45 degrés. Cet angle solide correspond au cône de mesure CE de l'organe émetteur, qui est ici un cône d'émission CE (figure 2). Ce cône d'émission CE est délimité sur la figure 6 par les flèches 155 représentant les ultrasons émis. Il est centré autour de l'axe de mesure AME qui constitue une direction d'émission d'ultrasons privilégiée de l'organe émetteur.

Il n'est pas nécessaire que le positionnement de l'organe émetteur 150 par rapport à la tête 10 de l'individu soit ajusté de manière très précise, car le cône d'émission CE de l'émetteur 150 couvre un large cône de mesure et un positionnement approximatif de la tablette 110 devant les yeux 20 de l'individu assure que la tête 10 de l'individu entre dans ce cône d'émission au moins partiellement et donc reçoit des ultrasons qu'elle réfléchit.

Les organes récepteurs 151 utilisés présentent au contraire un cône de mesure CR (figure 2), centré sur l'axe de mesure AMR constituant la direction de réception des ultrasons privilégiée de chacun de ces organes récepteurs 151. Il s'agit dans ce cas d'un cône de réception CR d'angle au sommet inférieur à 45 degrés.

Chaque organe récepteur 151 comporte ici un élément récepteur 151A placé dans un corps présentant la forme d'un orifice cylindrique 152 creusé dans la face avant du dispositif de mesure 100. Plus précisément, les orifices cylindriques 152 sont ici creusés dans la face avant du support de mesure 130, comme représenté sur la figure 2.

Les organes récepteurs 151 ne reçoivent ainsi que les ultrasons réfléchis par la tête 10 de l'individu et plus particulièrement par la monture 30 disposée sur sa tête dans une direction très proche de l'axe longitudinal de l'orifice cylindrique 152 (figure 2), qui représente ici l'axe de mesure AMR de ces organes récepteurs 151, ici perpendiculaire à la face avant 132 du support de mesure 130. C'est le cas par exemple des ultrasons représentés par les flèches 156 sur la figure 2.

Le cône de réception CR de l'organe récepteur 151 est ici limité par le contour de l'orifice cylindrique 152, quel que soit le cône de réception intrinsèque de l'élément récepteur utilisé.

Dans l'exemple représenté sur les figures 1, 2 et 5, les axes de mesure AMR, AME des organes récepteurs 151 et émetteur 150 du dispositif de mesure 100 sont parallèles entre eux et perpendiculaires à la face avant 132 du support de mesure 130.

Ces organes récepteurs et émetteurs sont montés sur la tablette 110 pour pivoter conjointement par rapport à la partie d'affichage 120 et forment donc tous le même angle GAMMA1 avec le plan de la partie d'affichage 120 qui peut être modifié par pivotement du support de mesure 130 au moins dans la plage angulaire précitée d'inclinaison du dispositif de mesure 100.

En variante, on peut envisager que ces axes de mesure ne soit pas parallèles entre eux. Leur inclinaison peut permettre de prendre en compte une forte courbure de la monture de lunettes portée par l'individu.

Les ultrasons réfléchis selon l'axe de mesure AMR de chaque organe récepteur 151 proviennent d'une surface sensiblement perpendiculaire à l'axe longitudinal de l'orifice cylindrique 152. C'est pourquoi il est donc ici particulièrement important d'incliner les organes récepteurs 151 de telle sorte que l'axe longitudinal de l'orifice cylindrique 152, confondu avec l'axe de mesure AMR de l'organe récepteur 151 correspondant, soit sensiblement perpendiculaire au plan moyen PM de la monture 30 de lunettes de l'individu (figures 1, 2 et 5).

Ici cela correspond à une position angulaire du support de mesure 130 dans laquelle la face avant 132 de ce support de mesure 130 est sensiblement parallèle au plan moyen PM de la monture 30.

Comme mentionné précédemment, en première approximation, le plan moyen PM de la monture 30 est confondu avec le plan de regard PR perpendiculaire à la direction du regard DR. En pratique, il ne s'en écarte que de quelques degrés et cette approximation ne conduit qu'à une faible imprécision de la mesure de distance.

Il s'agit donc ici de placer le support de mesure 130 dans une position angulaire dans laquelle il est perpendiculaire à la direction de regard DR.

Alternativement, on peut prévoir que les organes récepteurs présentent un cône de réception large et que l'émetteur présente un cône d'émission étroit.

Afin de déterminer la position angulaire du support de mesure 130 autour de l'axe de pivotement X, qui est adapté à la détection des ultrasons réfléchis sur la monture 30 de lunettes de l'individu, le dispositif comporte des moyens de détection visuelle 160 permettant à l'individu de détecter visuellement une position relative de sa tête 10 et des organes récepteurs 151 dans laquelle le plan moyen de la monture PM se trouve sensiblement perpendiculaire à l'axe de mesure AMR des organes récepteurs 151 pour détecter un signal d'intensité maximum ou au moins supérieur à un seuil.

Ce réglage assure alors que sa tête 10 est au moins partiellement située dans le cône de réception CR des organes récepteurs 151 (figure 2), pour une position de lecture déterminée de la tablette 110 par rapport à la tête de l'individu.

Comme représenté sur la figure 1, ces moyens de détection visuelle 160 comportent au moins une cible 161 solidaire des organes récepteurs 151. Cette cible 161 consiste ici en un cercle 161 qui se trouve dans l'épaisseur du support de mesure 130, dans un plan parallèle à la face avant 132 de ce support de mesure 130 orientée vers l'individu, en retrait par rapport à cette face avant 132.

Cette cible 161 se situe en regard de l'organe émetteur 150 porté par la face avant 132 du support de mesure 130.

Le support de mesure 130 est de préférence réalisé dans un matériau transparent, par exemple un matériau plastique transparent, au moins pour l'épaisseur comprise entre ladite face avant 132 et la cible 161.

La cible 161 est disposée de manière à ce que, lorsque le support de mesure 130 est regardé selon une direction de regard DR perpendiculaire à sa face avant 132, l'émetteur 150, qui exerce alors une fonction de marquage, apparaît situé au centre du cercle de la cible 161.

Lorsque le support de mesure 130 est regardé selon une direction de regard DR non perpendiculaire à sa face avant, l'émetteur 150 apparaît excentré par rapport au cercle de la cible 161, car celle-ci est située dans un plan différent de celui de l'émetteur 150. Ceci est du à un phénomène de parallaxe.

Une fois que l'individu a placé la partie d'affichage 120 de la tablette 110 dans sa position de lecture, il lui est alors possible de regarder la position relative de l'émetteur 150 et du cercle formant la cible 161 pour déterminer si la face avant 132 du support de mesure 130 est perpendiculaire à sa direction de regard DR.

Si ce n'est pas le cas, l'individu fait varier la position angulaire du support de mesure 130 par rapport à la partie d'affichage 120 de la tablette autour de l'axe de pivotement X en actionnant manuellement la molette 131.

Lorsque l'individu voit l'émetteur 150 centré dans la cible 161, il stoppe le pivotement du support de mesure 130 et laisse ce support de mesure 130 dans cette position angulaire, qui permettra la prise de mesure. L'axe longitudinal de l'orifice cylindrique 152 est alors sensiblement perpendiculaire au plan moyen de la monture PM, ce qui assure une position adaptée des organes récepteurs 151 directifs par rapport aux lunettes 30 de l'individu pour la prise de mesure.

Alternativement, si les organes récepteurs sont directifs sans être placés au fond d'un orifice cylindrique, c'est la bissectrice du cône de réception de ces organes récepteurs qui joue le rôle de l'axe de mesure AMR des organes récepteurs et qui est orientée perpendiculairement au plan moyen de la monture PM.

En variante, le pivotement du support de mesure peut être motorisé et commandé par une unité de pilotage électronique. L'individu déclenche alors le balayage de toutes les positions angulaires possibles du support de mesure et l'arrête lorsque le centrage de la cible autour de l'émetteur est réalisé.

En variante encore, le support de mesure peut être monté sur la tablette par l'intermédiaire d'une charnière, et le balayage est réalisé soit manuellement par l'individu qui fait pivoter ce support en agissant directement sur lui, soit automatiquement si le pivotement du support est motorisé. Il n'est pas alors prévu de molette.

En variante, le dispositif peut comporter des marquages distincts des organes émetteurs et récepteurs.

Le principe du réglage de la position angulaire est alors représenté sur la figure 7 et les figures 4A, 4B, 4C qui illustrent différentes variantes de formes de cibles et marquages formant des moyens de détection visuelle. Les marquages 260; 560; 760 sont situés dans un premier plan et les cibles 360; 460; 660 sont disposées dans un second plan parallèle au premier plan et distinct de ce dernier, les premier et second plans étant situés à des distances H1, H2 différentes de la tête de l'individu suivant la direction de regard DR (figure 3). Elle apparaissent centrées l'une par rapport à l'autre à l'individu lorsque la direction de regard est perpendiculaire aux plans les contenant (figures 4A, 4B, 4C).

Les figures 4A, 4B, 4C montrent différents couples de cibles et marquages pouvant être utilisés : cercle 260 au centre d'un carré 360, cercle 560 entre deux lignes parallèles 460, ou deux cercles 660, 760 concentriques.

En variante encore, la cible des moyens de détection visuelle peut être placée au fond d'une cavité ménagée dans la face avant du support de mesure de telle sorte qu'elle ne soit visible par l'individu que lorsque le support de mesure se trouve dans une position angulaire dans laquelle celui-ci est perpendiculaire à la direction de regard de l'individu. Cette cible est alors de préférence lumineuse, telle une diode électroluminescente.

En variante, le dispositif de mesure peut comporter des moyens de détection automatique pour détecter automatiquement une position relative de la tête de l'individu et des organes récepteurs dans laquelle les axes de mesure des organes émetteurs et récepteurs sont sensiblement perpendiculaires au plan moyen de la monture PM pour une position de lecture déterminée. La tête de l'individu est alors automatiquement au moins partiellement située dans le cône de réception de ces organes récepteurs pour cette position de lecture.

Les moyens de détection automatique comportent alors par exemple des moyens de balayage d'une plage de positions angulaires possibles des organes récepteurs par rapport à la partie d'affichage du support de lecture, pendant l'émission d'ultrasons, pour que l'axe de mesure de ces organes récepteurs balaye la tête 10 de l'individu. La plage de positions angulaires balayée couvre au moins 15 degrés vers l'avant ou vers l'arrière de la tablette par rapport à une position angulaire moyenne de référence dans laquelle l'angle GAMMA1 entre l'axe de mesure de l'organe récepteur et la partie d'affichage est compris entre 10 et 80 degrés.

Ce balayage peut être réalisé manuellement par l'individu qui tourne la molette du dispositif. Il peut également être réalisé de manière automatique : le pivotement du support de mesure est alors motorisé et commandé par des moyens de pilotage électronique.

En variante, le support de mesure peut être monté sur la tablette par l'intermédiaire d'une charnière, et le balayage est réalisé soit manuellement par l'individu qui fait pivoter ce support en agissant directement sur lui, soit automatiquement si le pivotement du support est motorisé. Il n'est pas alors prévu de molette.

Un balayage automatique présente l'avantage de ne pas solliciter l'individu, qui peut continuer à lire les signes de la partie d'affichage 120. Cela limite le risque que l'individu modifie la position de la tablette 110 lors du réglage de la position angulaire du support de mesure 130.

La mesure est ainsi plus précise.

Lesdits moyens de traitement informatique et électronique des signaux d'ultrasons détectés par les organes récepteurs 151 traitent les signaux détectés en fonction de la position angulaire du support de mesure 130 et en déduisent la position angulaire recherchée.

En particulier, la position angulaire recherchée correspond à celle pour laquelle une intensité maximale de signaux ultrasons est détectée.

Alternativement, la position angulaire recherchée correspond à celle pour laquelle l'intensité du signal ultrasons est supérieure à un seuil.

Ce seuil peut correspondre par exemple à un pourcentage de l'intensité maximale du signal d'ultrasons obtenue lors d'une étape de calibration.

La durée d'émission d'ultrasons au bout de laquelle l'intensité maximale de signal est détectée est en générale inférieure ou égale à 40 secondes.

Les moyens de pilotage électroniques des moyens de balayage motorisés commandent alors le pivotement du support de mesure 130 vers cette position angulaire.

Pendant les étapes de pivotement du support de mesure 130 vers sa position angulaire dans laquelle les axes de mesure des organes émetteurs et récepteurs sont sensiblement perpendiculaires au plan moyen de la monture PM, la position relative de la tête 10 de l'individu et de la partie d'affichage 120 du dispositif de mesure 100 ne change pas : la tablette 110 reste dans la position de lecture prédéfinie, naturelle et non contrainte par la réalisation de la mesure.

Une fois que le support de mesure 130 est pivoté selon sa position angulaire recherchée, l'émission d'ultrasons est déclenchée et le signal d'ultrasons est détecté.

La durée d'émission et de détection des ultrasons est par exemple ici égale à 10 secondes.

Quel que soit le mode de réalisation du dispositif de mesure, les moyens de traitement informatique et électronique du dispositif de mesure 100 déterminent la distance caractéristique de lecture recherchée, à savoir ici la distance entre la monture 30 (comme dans l'exemple décrit) ou les lentilles (en variante analogue aisément transposable) des lunettes équipant l'individu et le dispositif de mesure 100. Lorsque l'organe émetteur-récepteur est disposé au fond d'un orifice conique, celui-ci joue un rôle de guide d'onde ce qui contribue à amplifier le signal reçu.

L'unité de traitement électronique est adaptée à extraire du signal d'ultrasons reçu par l'organe récepteur la partie de ce signal qui est réfléchie par les lentilles ou la monture des lunettes portées par l'individu, à l'exclusion de la partie de ce signal qui est réfléchie par la peau de l'individu.

L'intensité de signal reçu dépend de la nature du matériau ayant réfléchi le signal d'ultrasons émis et de l'écart entre la position angulaire de l'axe de mesure des organes émetteur-récepteur et la position angulaire de mesure dans laquelle cet axe de mesure est perpendiculaire au plan moyen de la monture.

L'amplitude absolue du signal reçu, c'est-à-dire son intensité permet de déduire si le signal reçu provient d'une réflexion sur la peau de l'individu ou d'une réflexion sur la monture de lunettes.

A titre d'exemple, pour une distance caractéristique de lecture d'environ 40 centimètres, le signal reçu après réflexion sur la monture est environ quatre fois plus intense que le signal reçu après réflexion sur la peau.

Il est ainsi possible de discriminer facilement le signal reçu après réflexion sur la monture de lunettes recherché du signal reçu après réflexion sur la peau, et ce même si la position angulaire de l'axe de mesure des organes émetteur-récepteur n'est pas exactement la position angulaire de mesure.

Les moyens de traitement informatique et électronique du dispositif de mesure réalisent un ajustement automatique du gain, c'est-à-dire de l'amplification du signal reçu par l'élément récepteur ou émetteur-récepteur, en fonction de l'amplitude du signal reçu.

Cette amplitude dépend notamment de la surface des lunettes portées par l'individu. Une petite monture de lunettes donnera un signal reçu d'amplitude plus faible qu'une monture de lunettes de plus grande taille.

L'augmentation de l'amplification de ce signal par les moyens de traitement informatique et électronique du dispositif de mesure permet de compenser cette différence.

En outre, en cas de pic de signal très intense, d'amplitude supérieure à une première valeur seuil prédéfinie, les moyens de traitement informatique et électronique sont programmés pour procéder à un écrêtement du signal avant de réaliser l'ajustement du gain.

Les moyens de traitement informatique et électronique du dispositif de mesure détectent le signal reçu dont l'intensité est supérieure à une deuxième valeur seuil prédéterminée. Cette deuxième valeur seuil correspond de préférence à une valeur d'intensité du signal assurant que le signal reçu provient d'une réflexion du signal émis sur la monture de lunettes de l'individu.

Cette deuxième valeur seuil est déterminée à partir de mesures d'étalonnage réalisées au préalable ou correspond à une valeur standard prédéterminée par rapport au signal maximal attendu pour une monture de taille donnée, et pour un gain déterminé. Cette valeur standard est par exemple prise égale à 20 pour cent de ce signal maximal.

Plusieurs valeurs seuils inférieures à ladite deuxième valeur seuil peuvent être prévues pour prendre en compte la diminution de l'intensité du signal reçu en fonction de l'écart entre la position angulaire de l'axe de mesure des organes émetteur-récepteur et la position angulaire de mesure.

Ces différentes valeurs seuils sont détectées successivement par les moyens de traitement informatique et électronique du dispositif de mesure en cas d'échec de détection d'un signal au-dessus de la valeur seuil la plus élevée. Les valeurs seuil sont détectées dans un ordre décroissant jusqu'à ce qu'un signal soit détecté.

Ce mode de seuillage permet d'éviter les échecs de détection, qui obligeraient à réaliser une nouvelle mesure.

Bien entendu, si le signal reçu présente une intensité inférieure à la valeur seuil la plus basse, le dispositif de mesure indique par un message d'erreur que la mesure doit être réitérée.

Le signal reçu en fonction du temps présente dans un cas idéal où l'élément récepteur ne recevrait que le signal réfléchi par les lunettes de l'individu une forme carrée.

En réalité, le signal d'ultrasons reçu après réflexion sur la tête du porteur comporte une composante aux temps les plus courts correspondant au signal réfléchi par la peau de l'individu située en avant des lunettes, puis une composante à des temps intermédiaires correspondant au signal réfléchi par les lunettes de l'individu et enfin une composante aux temps longs correspondant au signal réfléchi par la peau de l'individu située en arrière des lunettes par rapport au dispositif de mesure.

Le signal reçu par les organes récepteurs ou émetteur-récepteur présente alors une forme approchée aplatie qui est liée à la texture de la surface sur laquelle les ultrasons sont réfléchis.

Les moyens de traitement du signal reçu procèdent ainsi à une étape de correction de la pente du signal reçu réel correspondant à une amplification de cette pente afin de se rapprocher du cas idéal et déterminer précisément le moment auquel le signal réfléchi sur les lunettes de l'individu est reçu.

Ce moment est par exemple déterminé comme le temps correspondant à un seuil paramétrable (typiquement situé entre la mi-hauteur et 20% de la hauteur) de la pente ascendante du signal reçu.

Une première valeur de la distance caractéristique de lecture séparant les lunettes de l'individu et les organes émetteur et récepteur du dispositif de mesure est alors déterminée en fonction de ce temps.

De préférence, une fois une première valeur de la distance recherchée déterminée, le signal reçu subit également une étape de correction d'étalonnage correspondant à la soustraction d'un signal de référence correspondant au signal reçu lorsque les ultrasons sont réfléchis par la peau.

Ce signal de référence correspond au signal reçu par l'organe récepteur ou émetteur-récepteur par réflexion sur la peau du visage d'un individu de référence moyen lorsque cet individu de référence se trouve à une distance du dispositif de mesure égale à la première valeur de la distance caractéristique de lecture déterminée.

Ce signal de référence est par exemple déterminé en réalisant trois mesures d'étalonnage à des distances caractéristiques de lecture connues, par exemple pour des distances caractéristiques de lecture de 30, 40 et 50 centimètres.

Une interpolation linéaire de ces signaux permet d'en déduire un signal de référence pour toutes les distances caractéristiques de lecture comprises entre 30 et 50 centimètres.

Les moyens de traitement informatiques et électroniques du signal soustraient au signal reçu le signal de référence correspondant à la première valeur de la distance caractéristique de lecture déterminée.

Les étapes précédentes sont ensuite répétées sur ce signal corrigé afin de déterminer une deuxième valeur de la distance caractéristique de lecture plus précise que la première.

La précision de la mesure ainsi réalisée est d'environ 1 à 2 centimètres.

La durée totale de la mesure est de préférence inférieure ou égale à 1 minute et 15 secondes. Cette mesure est donc réalisée très rapidement.

Plus particulièrement ici, une première distance caractéristique de lecture recherchée est déterminée à partir de la moyenne des signaux d'ultrasons détectés par les deux organes récepteurs 151 du sous-ensemble de mesure 140 et traités par les moyens de traitement informatiques et électroniques comme expliqués précédemment.

Ces deux organes récepteurs 151 du sous-ensemble de mesure 140 sont disposés chacune en regard de l'une des lentilles ophtalmiques de la monture 30 de lunettes de l'individu, et donne chacun une mesure de la distance caractéristique entre l'une des lentilles ophtalmiques de cette monture 30 et l'organe récepteur 151 correspondant.

La première distance moyenne caractéristique correspond donc à une moyenne des distances entre chaque lentille et le dispositif de mesure.

La réalisation de cette moyenne permet de limiter l'influence sur la mesure d'une faible rotation de la tête autour d'un axe verticale : cela limite l'erreur introduite par le fait que le plan sagittal PSAG de la tête du porteur n'est pas parfaitement confondu avec le plan de symétrie PS du dispositif de mesure.

De préférence, les mesures sont répétées et moyennées : la distance caractéristique de lecture recherchée est déterminée à partir de la moyenne d'au moins cinq premières distances caractéristiques ainsi déterminées.

En variante, les moyens de traitement informatique et électronique déterminent deux distances caractéristiques à partir des signaux d'ultrasons reçus par chacun des deux organes récepteurs qui correspondent respectivement à la mesure de la distance séparant chaque lentille ophtalmique de la tablette. Les moyens de traitement informatique et électronique font ensuite la moyenne de ces deux distances pour déterminer la distance caractéristique de lecture recherchée. Plusieurs de ces valeurs peuvent ensuite être moyennées pour obtenir une distance caractéristique de lecture moyenne plus précise.

Le dispositif de mesure 100 comporte en outre éventuellement des moyens de transmission de la mesure de distance réalisée, avec ou sans fil, vers un ou plusieurs autres dispositifs utilisés par l'opticien.

Le dispositif de mesure 100 peut en particulier être une unité de mesure faisant partie d'un ensemble de mesure plus important.

Le dispositif de mesure 100 peut également comporter les moyens d'affichage de la distance caractéristique de lecture déterminée. Celle-ci peut alors être lue directement sur le dispositif de mesure par l'individu ou par l'opticien.

Le dispositif de mesure 100 comporte de préférence des moyens d'alimentation autonomes, par exemple des piles ou une batterie rechargeable. La présente invention n'est nullement limitée aux modes de réalisation décrits et représentés mais l'homme du métier saura y apporter toute variante conforme à son esprit.

Le dispositif de mesure peut par exemple comporter des instruments de mesure additionnels embarqués, par exemple un inclinomètre adapté à mesurer l'angle d'inclinaison entre le plan de la partie d'affichage du dispositif de mesure et un plan horizontal. Cette inclinaison peut être prise en compte pour améliorer la précision des mesures de distance réalisées.

Les différentes variantes décrites peuvent être combinées. En particulier, chaque variantes de réalisation envisagées dans la description dans le cas d'un organe émetteur présentant un cône d'émission large et de deux organes récepteurs présentant un cône de réception étroit peut être envisagée pour un organe émetteur comportant un cône d'émission étroit et des organes récepteurs présentant un cône de réception large.

On peut envisager que le sous-ensemble de mesure puisse pivoter selon plus d'une direction : le support de mesure serait alors monté sur le support de lecture par l'intermédiaire d'une rotule.

On peut envisager que l'organe de mesure, récepteur ou émetteur, soit monté mobile directement sur la tablette, sans support de mesure intermédiaire. Il est alors par exemple motorisé et orientable par rapport à la tablette.

On peut également envisager que la partie d'affichage du support de lecture soit constituée par un dispositif d'affichage amovible. Ce dispositif d'affichage amovible est par exemple un téléphone portable ou une tablette multimédia qui peut être fournie par l'opticien ou propre à l'individu. Le support de lecture comporte alors une station d'accueil de ce dispositif d'affichage amovible.

D'autres moyens de détection visuelle de l'inclinaison de mesure du dispositif de mesure peuvent être envisagés. En particulier, il est possible par exemple de prévoir que ces moyens de détection visuelle soient liés aux signes affichés par la partie d'affiche du dispositif de mesure. Ces signes ne sont alors visibles par l'individu que sous une incidence dans laquelle le dispositif de mesure est orienté selon l'inclinaison de mesure.

## Revendications

1. Dispositif de mesure d'une distance caractéristique de lecture d'un individu en posture naturelle de vision de près, comportant :
- un support de lecture (110) portable ou librement mobile, présentant une partie d'affichage (120) plane adaptée à l'affichage de signes,
- un sous-ensemble de mesure (140) comportant au moins un organe émetteur (150) et au moins un organe récepteur (151) d'ultrasons, portés par le support de lecture (110), l'un au moins de ces organes, appelé premier organe de mesure(150, 151), possédant un axe de mesure (AME, AMR), selon lequel l'émission ou la réception par le premier organe (151) de mesure est privilégiée, ledit axe de mesure étant inclinable par rapport au plan de la partie d'affichage au moins dans une plage angulaire comprise entre 10 et 80 degrés
**caractérisé en ce que** le premier organe de mesure (150, 151) est monté mobile sur le support de lecture (110) pour au moins pivoter par rapport au plan de ladite partie d'affichage (120) du support de lecture.

2. Dispositif (100) de mesure selon la revendication 1, dans lequel au moins un organe émetteur (150) et au moins un organe récepteur (151) sont montés pour pivoter conjointement sur le support de lecture (110).

3. Dispositif (100) de mesure selon l'une des revendications 1 et 2, dans lequel ledit premier organe de mesure (150, 151) présente un cône de mesure (CE, CR) centré sur son axe de mesure (AME, AMR), d'angle au sommet inférieur à 45 degrés.

4. Dispositif (100) de mesure selon la revendication 3, comportant des moyens de détection (160) visuelle permettant à l'individu de détecter visuellement une position relative de sa tête (10) et dudit premier organe de mesure (150, 151) dans laquelle sa tête (10) est au moins partiellement située dans le cône de mesure (CE, CR) dudit premier organe de mesure (150, 151), pour une position relative déterminée de la tête (10) et du support de lecture (110).

5. Dispositif de mesure selon la revendication 3, comportant des moyens de détection automatique pour détecter automatiquement une position relative de la tête de l'individu et dudit premier organe de mesure dans laquelle sa tête est au moins partiellement située dans le cône de mesure dudit premier organe de mesure, pour une position relative déterminée de la tête et du support de lecture.

6. Dispositif de mesure selon la revendication 5, dans lequel lesdits moyens de détection automatique comportent des moyens de balayage d'une plage de positions angulaires dudit premier organe de mesure par rapport à la partie d'affichage du support de lecture, pour que l'axe de mesure du premier organe de mesure balaye la tête de l'individu.

7. Dispositif de mesure selon l'une des revendications 1 à 6, comportant une unité de traitement électronique est adaptée à extraire du signal d'ultrasons reçu par l'organe récepteur la partie de ce signal qui est réfléchie par les lentilles ou la monture de lunettes portées par l'individu, à l'exclusion de la partie de ce signal qui est réfléchie par la peau de l'individu.

8. Procédé de mesure d'une distance caractéristique de lecture d'un individu en posture naturelle de vision de près comportant les étapes suivantes:
a2) fourniture à l'individu d'un dispositif de mesure (100) comportant :
- un support de lecture (110) portable ou librement mobile, présentant une partie d'affichage (120) plane adaptée à l'affichage de signes,
- un sous-ensemble de mesure (140) comportant au moins un organe émetteur (150) et au moins un organe récepteur (151) d'ultrasons portés par le support de lecture, l'un au moins de ces organes (150, 151), appelé premier organe de mesure (150, 151), monté sur le support de lecture (110) pour au moins pivoter par rapport à ladite partie d'affichage (120) du support de lecture (110) et possédant un axe de mesure (AME, AMR), selon lequel l'émission ou la réception par le premier organe de mesure (150, 151) est privilégiée,
b2) positionnement de la partie d'affichage (120) du support de lecture (110) par l'individu dans une position de lecture lui convenant afin de lire les signes de la partie d'affichage (120),
c2) dans cette position de lecture fixée selon l'étape b2), on fait pivoter ledit premier organe de mesure (150, 151) par rapport à la partie d'affichage (120) pour placer ledit premier organe de mesure (150, 151) dans une position angulaire pour laquelle l'organe récepteur (151) capte, avec une intensité maximum ou supérieure à un seuil, les ultrasons issus de l'organe émetteur (150) et réfléchis par la tête (10) de l'individu ou par un accessoire (30) rapporté sur la tête,
d2) dans la configuration définie aux étapes b2) et c2) on détermine la distance caractéristique (D) recherchée en fonction du signal délivré par l'organe récepteur (151) et représentatif du signal d'ultrasons issu de l'organe émetteur (150) et réfléchi.

9. Procédé de mesure selon la revendication 8, selon lequel, à l'étape c2), ledit premier organe de mesure (150, 151) présentant un cône de mesure (CE, CR) d'angle au sommet inférieur à 45 degrés, on fait pivoter ledit premier organe de mesure (150, 151) par rapport à la partie d'affichage (120) pour le placer dans une position angulaire pour laquelle la tête (10) de l'individu est au moins partiellement située dans le cône de mesure (CE, CR) du premier organe de mesure (150, 151).

10. Procédé de mesure selon l'une des revendications 8 et 9, selon lequel, à l'étape c2), le dispositif (100) comportant au moins une cible (161) et un marquage (150) solidaires dudit premier organe de mesure (150, 151), le marquage (150) est la cible (161) étant décalés suivant la direction de l'axe de mesure (AME, AMR) du premier organe de mesure, l'individu fait pivoter le premier organe de mesure (150, 151) de manière à réaliser le centrage visuel de ladite cible (161) sur ledit marquage (150).

11. Procédé de mesure selon l'une des revendications 8 et 9, selon lequel le dispositif comportant au moins une cible solidaire dudit premier organe de mesure et disposée au fond d'une cavité de telle sorte que la cible ne soit visible par l'individu que dans une plage angulaire prédéterminée d'orientation de l'organe de mesure par rapport à la tête de l'individu et dans lequel, à l'étape c2), l'individu fait pivoter le premier organe de mesure de manière à voir la cible.

12. Procédé de mesure selon l'une des revendications 8 à 11, selon lequel, à l'étape c2),
- on commande l'émission d'ultrasons par l'organe émetteur (150) d'ultrasons,
- on balaye manuellement ou automatiquement les différentes positions angulaires possibles du premier organe de mesure (150, 151) pendant l'émission d'ultrasons,
- on détecte un signal d'ultrasons émis et réfléchis par la tête (10) de l'individu ou par un accessoire (30) rapporté sur la tête pendant ce balayage,
- on détermine, en fonction de ce signal détecté, la position angulaire du premier organe de mesure (150, 151) recherchée, pour laquelle l'organe récepteur (151) capte les ultrasons issus de l'organe émetteur (150) et réfléchis.

13. Procédé de mesure selon l'une des revendications 8 à 12, selon lequel, à l'étape c2), on fait pivoter le premier organe de mesure (150, 151) par rapport à la partie d'affichage (120) du support de lecture (110) autour d'un axe horizontal (X) situé dans le plan moyen du support de lecture (110).

## Patentansprüche

1. Vorrichtung zum Messen eines charakteristischen Leseabstands einer Person in natürlicher Nahsichthaltung, die aufweist:
- einen tragbaren oder frei beweglichen Leseträger (110), der einen ebenen Anzeigeteil (120) aufweist, der für die Anzeige von Zeichen geeignet ist,
- eine Messbaugruppe (140), die mindestens ein Ultraschall-Emitterelement (150) und mindestens ein Ultraschall-Empfangselement (151) aufweist, die vom Leseträger (110) getragen werden, wobei mindestens eines dieser Elemente, erstes Messelement (150, 151) genannt, eine Messachse (AME, AMR) besitzt, gemäß der die Emission oder der Empfang durch das erste Messelement (151) bevorzugt ist,
wobei die Messachse bezüglich der Ebene des Anzeigeteils mindestens in einem Winkelbereich zwischen 10 und 80 Grad verstellbar ist,
**dadurch gekennzeichnet, dass** das erste Messelement (150, 151) beweglich auf den Leseträger (110) montiert ist, um zumindest bezüglich der Ebene des Anzeigeteils (120) des Leseträgers schwenken zu können.

2. Messvorrichtung (100) nach Anspruch 1, wobei mindestens ein Emitterelement (150) und mindestens ein Empfangselement (151) montiert sind, um zusammen auf dem Leseträger (110) zu schwenken.

3. Messvorrichtung (100) nach einem der Ansprüche 1 und 2, wobei das erste Messelement (150, 151) einen auf seine Messachse (AME, AMR) zentrierten Messkegel (CE, CR) mit einem Spitzenwinkel kleiner als 45 Grad aufweist.

4. Messvorrichtung (100) nach Anspruch 3, die Einrichtungen zur visuellen Erfassung (160) aufweist, die es der Person erlauben, eine relative Stellung ihres Kopfes (10) und des ersten Messelements (150, 151) visuell zu erfassen, in der ihr Kopf (10) sich zumindest zum Teil im Messkegel (CE, CR) des ersten Messelements (150, 151) befindet, für eine bestimmte relative Stellung des Kopfes (10) und des Leseträgers (110) .

5. Messvorrichtung nach Anspruch 3, die Einrichtungen zur automatischen Erfassung einer relativen Stellung des Kopfes der Person und des ersten Messelements aufweist, in der ihr Kopf sich zumindest zum Teil im Messkegel des ersten Messelements befindet, für eine bestimmte relative Stellung des Kopfes und des Leseträgers.

6. Messvorrichtung nach Anspruch 5, wobei die Einrichtungen zur automatischen Erfassung Einrichtungen zum Abtasten eines Bereichs von Winkelstellungen des ersten Messelements bezüglich des Anzeigeteils des Leseträgers aufweisen, damit die Messachse des ersten Messelements den Kopf der Person abtastet.

7. Messvorrichtung nach einem der Ansprüche 1 bis 6, die eine elektronische Verarbeitungseinheit aufweist, die geeignet ist, aus dem vom Empfangselement empfangenen Ultraschallsignal den Teil dieses Signals zu entnehmen, der von den von der Person getragenen Linsen oder Brillengestell reflektiert wird, mit Ausnahme des Teils dieses Signals, der von der Haut der Person reflektiert wird.

8. Verfahren zum Messen eines charakteristischen Leseabstands einer Person in natürlicher Nahsichthaltung, das die folgenden Schritte aufweist:
a2)Bereitstellen für die Person einer Messvorrichtung (100), die aufweist:
- einen tragbaren oder frei beweglichen Leseträger (110), der einen ebenen Anzeigeteil (120) aufweist, der für die Anzeige von Zeichen geeignet ist,
- eine Messbaugruppe (140), die mindestens ein Ultraschall-Emitterelement (150) und mindestens ein Ultraschall-Empfangselement (151) aufweist, die vom Leseträger (110) getragen werden, wobei mindestens eines dieser Elemente (150, 151), erstes Messelement (150, 151) genannt, auf den Leseträger (110) montiert ist, um mindestens bezüglich des Anzeigeteils (120) des Leseträgers (110) zu schwenken, und eine Messachse (AME, AMR) besitzt, gemäß der die Emission oder der Empfang durch das erste Messelement (150, 151) bevorzugt ist,
b2)Positionieren des Anzeigeteils (120) des Leseträgers (110) durch die Person in einer ihr angenehmen Lesestellung, um die Zeichen des Anzeigeteils (120) zu lesen,
c2)in dieser gemäß dem Schritt b2) festgelegten Stellung das erste Messelement (150, 151) bezüglich des Anzeigeteils (120) geschwenkt wird, um das erste Messelement (150, 151) in einer Winkelstellung anzuordnen, in der das Empfangselement (151) mit einer maximalen oder höher als eine Schwelle liegenden Intensität die Ultraschalle auffängt, die vom Emitterelement (150) stammen und vom Kopf (10) der Person oder von einem auf den Kopf aufgesetzten Zubehörteil (30) reflektiert werden,
d2)in der in den Schritten b2) und c2) definierten Konfiguration der gesuchte charakteristische Abstand (D) bestimmt wird, abhängig von dem Signal, das vom Empfangselement (151) geliefert wird und für das vom Emitterelement (150) stammende und reflektierte Ultraschallsignal repräsentativ ist.

9. Messverfahren nach Anspruch 8, wobei, im Schritt c2), wenn das erste Messelement (150, 151) einen Messkegel (CE, CR) mit einem Spitzenwinkel geringer als 45 Grad aufweist, das erste Messelement (150, 151) bezüglich des Anzeigeteils (120) geschwenkt wird, um es in eine Winkelstellung zu bringen, in der der Kopf (10) der Person sich zumindest zum Teil im Messkegel (CE, CR) des ersten Messelements (150, 151) befindet.

10. Messverfahren nach einem der Ansprüche 8 und 9, wobei, im Schritt c2), wenn die Vorrichtung (100) mindestens ein Ziel (161) und eine Markierung (150) aufweist, die fest mit dem ersten Messelement (150, 151) verbunden sind, wobei die Markierung (150) und das Ziel (161) gemäß der Richtung der Messachse (AME, AMR) des ersten Messelements versetzt sind, die Person das erste Messelement (150, 151) so schwenkt, dass die visuelle Zentrierung des Ziels (161) auf die Markierung (150) durchgeführt wird.

11. Messverfahren nach einem der Ansprüche 8 und 9, wobei die Vorrichtung mindestens ein fest mit dem ersten Messelement verbundenes und am Boden eines Hohlraums so angeordnetes Ziel aufweist, dass das Ziel für die Person nur in einem vorbestimmten Ausrichtungswinkelbereich des Messelements bezüglich des Kopfs der Person sichtbar ist, und wobei im Schritt c2) die Person das erste Messelement so schwenkt, dass sie das Ziel sieht.

12. Messverfahren nach einem der Ansprüche 8 bis 11, wobei, im Schritt c2)
- die Ultraschallemission durch das Ultraschall-Emitterelement (150) gesteuert wird,
- die verschiedenen möglichen Winkelstellungen des ersten Messelements (150, 151) während der Ultraschallemission manuell oder automatisch abgetastet werden,
- ein emittiertes und vom Kopf (10) der Person oder von einem auf den Kopf aufgesetzten Zubehörteil (30) reflektiertes Ultraschallsignal während dieser Abtastung erfasst wird,
- abhängig von diesem erfassten Signal die gesuchte Winkelstellung des ersten Messelements (150, 151) bestimmt wird, für die das Empfangselement (151) die vom Emitterelement (150) stammenden und reflektierten Ultraschalle auffängt.

13. Messverfahren nach einem der Ansprüche 8 bis 12, wobei im Schritt c2) das erste Messelement (150, 151) bezüglich des Anzeigeteils (120) des Leseträgers (110) um eine waagrechte Achse (X) geschwenkt wird, die sich in der Mittelebene des Leseträgers (110) befindet.

## Claims

1. A measurement device for measuring a characteristic reading distance of an individual in a natural posture for near vision, the device comprising:
• a portable or freely movable reading medium (110) presenting a plane display portion (120) suitable for displaying signs,
• a measurement subassembly (140) including at least one ultrasound emitter member (150) and at least one ultrasound receiver member (151),carried by the reading medium (110), at least one of said members, referred to as the first measurement member (150, 151) possessing a measurement axis (AME, AMR), along which emission or reception by the first measurement member (151) is privileged, said measurement axis being inclinable relative to the plane of the display portion at least over an angular range extending from 10 degrees to 80 degrees
**characterized in that** the first measurement member (150, 151) is movably mounted on the reading medium (110) to be capable at least of pivoting relative to the plane of said display portion (120) of the reading medium.

2. The measurement device (100) according to claim 1, wherein at least one emitter member (150) and at least one receiver member (151) are mounted to pivot together on the reading medium (110).

3. The measurement device (100) according to any one of claims 1 to 2, wherein said first measurement member (150, 151) presents a measurement cone (CE, CR) centered on its measurement axis (AME, AMR), the angle at the apex of the cone being less than 45 degrees.

4. The measurement device (100) according to claim 3, including visual detection means (160) enabling the individual to detect visually a relative position of said first measurement member (150, 151) and the head (10) of the individual in which the individual's head (10) is situated at least in part in the measurement cone (CE, CR) of said first measurement member (150, 151), in a determined relative position of the head (10) and the reading medium (110).

5. The measurement device according to claim 3, including automatic detection means for automatically detecting a relative position of said first measurement member and the head of the individual in which the individual's head is situated at least in part in the measurement cone of said first measurement member, in a determined relative position of the head and the reading medium.

6. The measurement device according to claim 5, wherein said automatic detection means include means for scanning a range of angular positions of said first measurement member relative to the display portion of the reading medium, so that the measurement axis of the first measurement member scans the individual's head.

7. The measurement device according to any one of claims 1 to 6, including an electronic processor unit that is suitable for extracting from the ultrasound signal received by the receiver member that portion of said signal that is reflected by the lenses or the frame of eyeglasses worn by the individual, to the exclusion of the portion of said signal that is reflected by the individual's skin.

8. A method of measuring a characteristic reading distance of an individual in a natural posture and in near vision, the method comprising the following steps:
a2) providing the individual with a measurement device (100) comprising:
• a portable or freely movable reading medium (110) presenting a plane display portion (120) suitable for displaying signs; and
• a measurement subassembly (140) including at least one ultrasound emitter member (150) and at least one ultrasound receiver member (151) carried by the reading medium, at least one of these members (150, 151), referred to as the first measurement member (150, 151) being mounted on the reading medium (110) to be capable at least of pivoting relative to said display portion (120) of the reading medium (110) and possessing a measurement axis (AME, AMR) along which the emission or the reception by the first measurement member (150, 151), is privileged;
b2) the individual positioning the display portion (120) of the reading medium (110) in a suitable reading position enabling the individual to read the signs of the display portion (120);
c2) in this reading position set in step b2), causing said first measurement member (150, 151) to pivot relative to the display portion (120) to place said first measurement member (150, 151) in an angular position in which the receiver member (151) picks up, with maximum intensity or intensity greater than a threshold, the ultrasound emitted by the emitter member (150) and reflected by the individual's head (10) or by an accessory (30) worn by the head; and
d2) in the configuration defined in steps b2) and c2), determining the looked-for characteristic distance (D) as a function of the signal delivered by the receiver member (151) and representative of the ultrasound signal emitted by the emitter member (150) and reflected.

9. The measurement method according to claim 8, wherein, in step c2), for said first measurement member (150, 151) presenting a measurement cone (CE, CR) having an angle at the apex of less than 45 degrees, said first measurement member (150, 151) is caused to pivot relative to the display portion (120) to place it in an angular position in which the individual's head (10) is situated at least in part in the measurement cone (CE, CR) of the first measurement member (150, 151).

10. The measurement method according to any one of claims 8 to 9, wherein in step c2), for the device (100) including at least one target (161) and at least one mark (150) secured to said first measurement member (150, 151), the mark (150) and the target (161) being offset along the direction of the measurement axis (AME, AMR) of the first measurement member, the individual causes the first measurement member (150, 151) to pivot in such a manner as to center said target (161) on said mark (150) visually.

11. The measurement method according to any one of claims 8 to 9, wherein, for the device including at least one target secured to said first measurement member and located at the end of a cavity in such a manner that the target is visible to the individual only over a predetermined range of orientation angles of the measurement member relative to the individual's head, in step c2) the individual causes the first measurement member to pivot in such a manner as to be able to see the target.

12. The measurement method according to any one of claims 8 to 11, wherein, in step c2), the following steps are performed:
• causing the ultrasound emitter member (150) to emit ultrasound;
• manually or automatically scanning the various possible angular positions of the first measurement member (150, 151) during ultrasound emission;
• detecting an ultrasound signal as emitted and reflected by the individual's head (10) or by an accessory (30) worn by the head during said scanning; and
• as a function of the detected signal, determining the looked-for angular position of the first measurement member (150, 151) in which the receiver member (151) picks up the ultrasound emitted by the emitter member (150) and reflected.

13. The measurement method according to any ones of claims 8 to 12, wherein, in step c2), the first measurement member (150, 151) is caused to pivot relative to the display portion (120) of the reading medium (110) about a horizontal axis (X) situated in the mean plane of the reading medium (110).
